# EUROPÄISCHE PATENTSCHRIFT

(11) **EP 1 480 620 B1**
(45) Veröffentlichungstag und Bekanntmachung des Hinweises auf die Patenterteilung: **22.11.2006**
(21) Anmeldenummer: 02791645.1
(22) Anmeldetag: 27.09.2002
(51) Int. Cl.: A61K 9/127

(54) **(ESTER)-LYSOLECITHINE IN LIPOSOMEN**
(ESTER)-LYSOLECITHINS IN LIPOSOMES
(ESTER)-LYSOLECITHINES DANS DES LIPOSOMEN

(30) Priorität: 28.09.2001 DE 10148065
(43) Veröffentlichungstag der Anmeldung: 01.12.2004
(73) Patentinhaber: MAX-PLANCK-GESELLSCHAFT ZUR FÖRDERUNG DER WISSENSCHAFTEN E.V., 80539 München (DE)
(72) Erfinder: EIBL, Jörg, 37120 Bovenden (DE)
(74) Vertreter: Dey, Michael
(86) Internationale Anmeldenummer: PCT/EP2002/010653
(87) Internationale Veröffentlichungsnummer: WO 2003/026617

(56) Entgegenhaltungen:
- EP-A- 0 158 880
- EP-A- 0 203 676
- WO-A-88/06439
- WO-A-94/26254
- US-A- 5 173 219
- US-B1- 6 193 997
- T.KITAGAWA, K. INOUE: "Properties of Liposomal Membranes Containing Lysolecithin" J.BIOCHEM., Bd. 79, Nr. 6, 1976, Seiten 1123-1133, XP009010918
- ITO M ET AL: "Rapid isolation and characterization of CHO mutants deficient in peroxisome biogenesis using the peroxisomal forms of fluorescent proteins" BIOCHIMICA ET BIOPHYSICA ACTA. MOLECULAR CELL RESEARCH, ELSEVIER SCIENCE PUBLISHERS, AMSTERDAM, NL, Bd. 1496, Nr. 2-3, 17. April 2000 (2000-04-17), Seiten 232-242, XP004278135 ISSN: 0167-4889
- BAMBAUER R ET AL: "Plasmapheresis in treatment of adrenoleukodystrophy." THERAPEUTIC APHERESIS: OFFICIAL JOURNAL OF THE INTERNATIONAL SOCIETY FOR APHERESIS AND THE JAPANESE SOCIETY FOR APHERESIS. UNITED STATES MAY 1997, Bd. 1, Nr. 2, Mai 1997 (1997-05), Seiten 152-154, XP009011157 ISSN: 1091-6660

## Beschreibung

Die vorliegende Erfindung betrifft neue Liposomzubereitungen, welche (Ester)Lysolecithinverbindungen enthalten. Die Liposomen sind insbesondere zur Verpackung von Wirkstoffen geeignet.

Zahlreiche Wirkstoffe, die an sich eine gute Wirksamkeit gegenüber verschiedensten Erkrankungen aufweisen, können in der Praxis nicht oder nur bedingt eingesetzt werden. Dies beruht oftmals darauf, dass Verbindungen eine schlechte Löslichkeit in wässrigen Lösungen aufweisen, was sie für eine intravenöse (I.V.) Verabreichung oder auch für eine orale Verabreichung in Form von Trinklösungen ungeeignet macht. Weiterhin werden viele pharmazeutisch wirksame Verbindungen bei oraler Verabreichung im Körper oftmals nur schlecht oder überhaupt nicht resorbiert. Weiterhin weisen viele pharmazeutisch wirksame Verbindungen bei Verabreichung in freier Form, insbesondere bei systemischer Verabreichung beträchtliche Nebenwirkungen auf, sodass sie nicht über längere Zeit oder/und in hohen Dosen verabreicht werden können.

Es wurden deshalb zahlreiche Versuche unternommen, Wirkstoffe in geeigneter Form zu "verpacken", um die oben genannten Nachteile zu überwinden. Eine häufig verwendete Verpackungsmethode ist dabei die Verwendung von Liposomen.

Trotzdem besteht, insbesondere auch aufgrund der Vielfältigkeit der einzukapselnden Wirkstoffe sowie der möglichen Verabreichungswege weiterhin ein großer Bedarf an Verpackungssystemen für therapeutisch wirksame Wirkstoffe.

Die Erfindung betrifft deshalb ein Liposom enthaltend
a) 10 bis 50 Mol-% (Ester)-Lysolecithin der Formel I 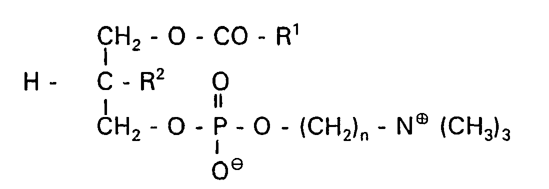 in der
   R¹ ein Kohlenwasserstoffrest mit 13 bis 23 C-Atomen ist,
   R² H, OH oder OR³ darstellt, worin R³ ein C₁-C₃-Alkyl- oder ein Allylrest ist, und
   n 2, 3 oder 4 bedeutet
b) 10 bis 50 Mol-% Cholesterin,
c) 10 bis 50 Mol-% Lecithin und
d) 5 bis 25 Mol-% eines negativen Ladungsträgers, ausgewählt aus der Gruppe bestehend aus Phosphatidylmono-, -di-, -tri- und -tetraglycerinen und Cholesterinphosphomono- und -oligoglycerinen.

Die Erfindung betrifft deshalb ein Liposom enthaltend
a) 10 bis 90 Mol-%, insbesondere 10 bis 50 Mol-% (Ester)-Lysolecithin der Formel 1 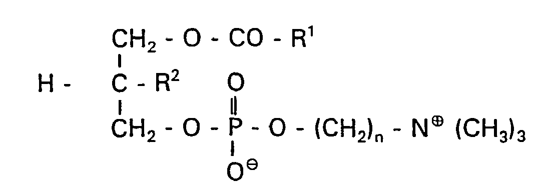 in der
   R¹ ein Kohlenwasserstoffrest mit 13 bis 23 C-Atomen ist,
   R² H, OH oder OR³ darstellt, worin R³ ein C₁-C₃-Alkyl- oder ein Allylrest ist, und
   n 2, 3 oder 4 bedeutet
b) 0 bis 60 Mol-%, insbesondere 10 bis 50 Mol-% Cholesterin,
c) 0 bis 50 Mol-%, insbesondere 10 bis 50 Mol-% Lecithin und
d) 3 bis 50 Mol-%, insbesondere 5 bis 25 Mol-% eines negativen Ladungsträgers, ausgewählt aus der Gruppe bestehend aus Phosphatidylmono-, -di-, -tri- und -tetraglycerinen, Cholesterinphosphomono- und -oligoglycerinen, Alkylphosphoglycerinen, Alkylphosphooligoglycerinen, Alkylphosphoglycolen, Alkylphosphopropandiolen-(1.3) oder/und Alkylphosphopropandiolen-(1.2).

Überraschenderweise wurde festgestellt, dass die erfindungsgemäßen Liposome mit der angegebenen Zusammensetzung hervorragende Eigenschaften als Wirkstoffträger aufweisen. Insbesondere konnte festgestellt werden, dass in den erfindungsgemäßen Liposomen verpackte Wirkstoffe eine deutlich überlegene Wirksamkeit gegenüber den entsprechenden freien Wirkstoffen aufweisen.

Weiterhin ist es möglich, verschiedenste Wirkstoffe auf einfache Weise, z.B. durch einfaches Zugeben oder Zumischen, in die erfindungsgemäßen Liposome einzubringen. Oftmals genügt einfaches Rühren zur Bildung von Liposomen mit eingekapseltem Wirkstoff, sodass drastische Verfahrensmaßnahmen nicht nötig sind. Weiterhin können die erfindungsgemäßen Liposome sterilfiltriert werden, z.B. durch Filter mit Porengrößen von 0,8 µm, 0,45 µm oder 0,2 µm. Die erfindungsgemäßen Liposome, insbesondere solche, die als Bestandteil c) ein Cholesterinphosphomono- oder -oligoglycerin enthalten, können auch hitzesterilisiert werden, insbesondere bei Temperaturen > 70 °C, > 80 °C, > 90 °C und bevorzugt > 95 °C. Die erfindungsgemäßen Liposome sind also hitzestabil. Sie sind weiterhin auch über einen großen pH-Bereich, z.B. von pH 3 bis pH 9, bevorzugt von pH 2 bis pH 10 stabil.

Die erfindungsgemäßen Liposome enthalten als Komponente a) ein Ester-Lysolecithin. Als Lysolecithine werden hierin auch Verbindungen bezeichnet, die keine freie OH-Gruppe aufweisen, sondern einen kurzkettigen, an den Sauerstoff gebundenen Kohlenwasserstoffrest, insbesondere einen C₁-C₃-Alkylrest oder Allylrest enthalten, da auch solche Verbindungen Lysolecithin-artige Eigenschaften aufweisen. In der Komponente a) kann der Kohlenwasserstoffrest R¹ 13 bis 23 C-Atome enthalten, bevorzugt sind insbesondere 15 bis 21 und stärker bevorzugt 16 bis 19 C-Atome. Besonders bevorzugt ist R¹ ein Alkylrest, insbesondere ein C₁₃-C₁₉-Alkylrest oder ein Alkenylrest, insbesondere ein C₁₅-C₂₃-Alkenylrest oder ein Alkadienylrest oder Alkatrienylrest, insbesondere ein C₁₅-C₂₃-Alkadienylrest oder C₁₅-C₂₃-Alkatrienylrest. Der Kohlenwasserstoffrest R¹ kann grundsätzlich gesättigt oder einfach oder mehrfach ungesättigt sein. Der Kohlenwasserstoffrest kann weiterhin verzweigt oder linear sein, wobei lineare Kohlenwasserstoffreste bevorzugt sind. Besonders bevorzugt ist R¹ ein Hexadecyl-, Heptadecyl-, Octadecyl-, Nonadecyl-, Eicosyl-, Hexadecenyl-, Heptadecenyl-, Octadecenyl-, Octadecadienyl-, Octadecatrienyl-, Nonadecenyl- oder Eicosenylrest.

R² in der Formel 1 ist bevorzugt H, OH oder OCH₃, besonders bevorzugt H oder OH.

Der polare Bestandteil der Verbindungen der Formel I besteht bevorzugt aus Phosphocholin (PC), d.h. n ist bevorzugt = 2.

Die Menge an Ester-Lysolecithinverbindung der Formel I in den erfindungsgemäßen Liposomen beträgt 10 bis 50 Mol-%, bevorzugt 20 bis 45 Mol-% und am meisten bevorzugt 25 bis 40 Mol-%.

Die Menge an Ester-Lysolecithinverbindung der Formel I in den erfindungsgemäßen Liposomen beträgt 10 bis 90 Mol-%, insbesondere 15 bis 90 Mol-%, bevorzugt 10 bis 50 Mol-%, mehr bevorzugt 20 bis 45 Mol-% und am meisten bevorzugt 25 bis 40 Mol-%.

Als weiteren Bestandteil enthalten die erfindungsgemäßen Liposomen Cholesterin (Bestandteil b)). Unter Cholesterin, wie hierin verwendet, werden Cholesterin selbst sowie Cholesterinderivate verstanden. Geeignete Cholesterinderivate sind beispielsweise Cholesterinoligoglycerine oder Cholesterinphosphocholin, wobei Cholesterinderivate mit einer hydrophilen Gruppe zur Verbesserung der Löslichkeit in wässrigen Medien bevorzugt sind.

Die Menge an Cholesterin in den erfindungsgemäßen Liposomen beträgt bevorzugt 20 bis 45 Mol-%, insbesondere 25 bis 40 Mol-%.

Die Menge an Cholesterin in den erfindungsgemäßen Liposomen beträgt 0 bis 60 Mol-%, bevorzugt 10 bis 50 Mol-%, mehr bevorzugt 20 bis 45 Mol-%, insbesondere 25 bis 40 Mol-%.

Als weiterer Bestandteil c) enthalten die erfindungsgemäßen Liposome Lecithin. Lecithine sind Glycerophospholipide, die sich aus Fettsäuren, Glycerin, Phosphorsäure und Cholin durch Veresterung bilden. Lecithine werden oftmals auch als Phosphatidylcholine (PC) bezeichnet. Erfindungsgemäß werden bevorzugt Lecithine der Formel 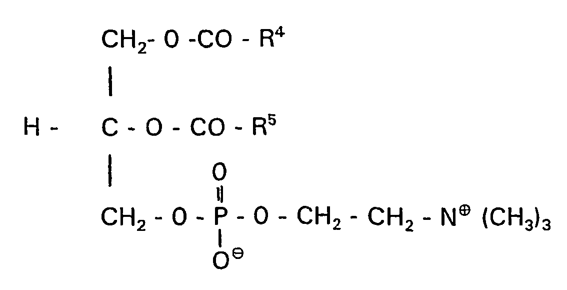 worin R⁴ und R⁵ jeweils unabhängig einen Kohlenwasserstoffrest mit 12 bis 30 C-Atomen, insbesondere mit 14 bis 24 C-Atomen darstellen, eingesetzt. Die Reste R⁴ und R⁵ können linear oder verzweigt und gesättigt oder einfach oder mehrfach ungesättigt sein. Bevorzugt handelt es sich bei den Resten R⁴ und R⁵ um Fettsäurereste.

Die Menge an Bestandteil c) in den erfindungsgemäßen Liposomen beträgt bevorzugt 20 bis 45 Mol-%, besonders bevorzugt 25 bis 40 Mol-%.

Die Menge an Bestandteil c) in den erfindungsgemäßen Liposomen beträgt O bis 50 Mol-%, insbesondere 0 bis 40 Mol-% oder 10 bis 50 Mol-%, bevorzugt 20 bis 45 Mol-%, besonders bevorzugt 25 bis 40 Mol-%.

Als weitere Komponente enthalten die erfindungsgemäßen Liposomen schließlich noch einen negativen Ladungsträger. Dieser Ladungsträger wird insbesondere ausgewählt aus Phosphatidylmonoglycerinen und Phosphatidyloligoglycerinen sowie Cholesterinphosphomonoglycerinen und Cholesterinphosphooligoglycerinen. Die Oligoglycerine weisen dabei bevorzugt 2 bis 4 Glycerinreste auf. Die Phosphatidyloligoglycerine sind, insbesondere in 1-sn- und 2-sn-Position mit Fettsäuren, welche gesättigt oder einfach oder mehrfach ungesättigt sein können und 12 bis 30 C-Atome, insbesondere 14 bis 26 C-Atome aufweisen können, verestert. Bevorzugt sind Phosphatidylglycerine mit Fettsäureresten, die eine cis-Doppelbindung aufweisen. Bevorzugt sind Phosphatidylglycerine, welche mindestens einen Oleylrest enthalten. Bevorzugte solche Verbindungen umfassen Dioleyl-Verbindungen, wie etwa Dioleyl-sn-glycero-3-phosphoglycerin, Dioleyl-sn-glycero-3-phosphodiglycerin, Dioleyl-sn-glycero-3-phosphotriglycerin sowie Dioleyl-sn-glycero-3-phosphotetraglycerin, welche bevorzugt als Na⁺-Salze eingesetzt werden. Es können auch Verbindungen mit zwei verschiedenen Resten, beispielsweise einem Oleylrest und einem Palmitoylrest eingesetzt werden. Die Ladung wird durch die am Phosphat vorhandenen negativen Ladungen beigetragen.

In einer weiteren bevorzugten Ausführungsform enthalten die erfindungsgemäßen Liposome als Bestandteil d) ein Cholesterinphosphoglycerin oder ein Cholesterinphosphooligoglycerin, insbesondere mit 1 bis 4 Glycerinresten. Überraschenderweise wurde festgestellt, dass unter Verwendung von Cholesterinphosphoglycerinen oder Cholesterinphosphooligoglycerinen Liposome erhalten werden können, die hitzestabil sind und somit hitzesterilisiert werden können. Dies stellt einen beträchtlichen Vorteil gegenüber vielen anderen Liposomformulierungen dar, insbesondere im Hinblick auf eine mögliche intravenöse oder subkutane Verabreichung der Liposome. Als Cholesterinphosphoglycerinverbindungen besonders bevorzugt sind:
1) Chol-phospho-glycerin, Na⁽⁺⁾-Salz 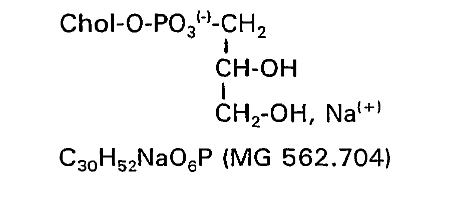
2) Chol-phospho-diglycerin, Na⁽⁺⁾-Salz 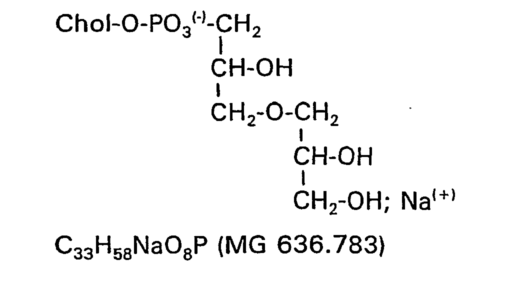
3) Chol-phospho-triglycerin, Na⁽⁺⁾-Salz 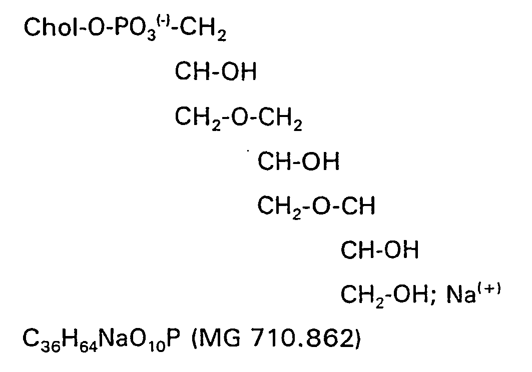
4) Chol-phospho-tetraglycerin, Na⁽⁺⁾-Salz

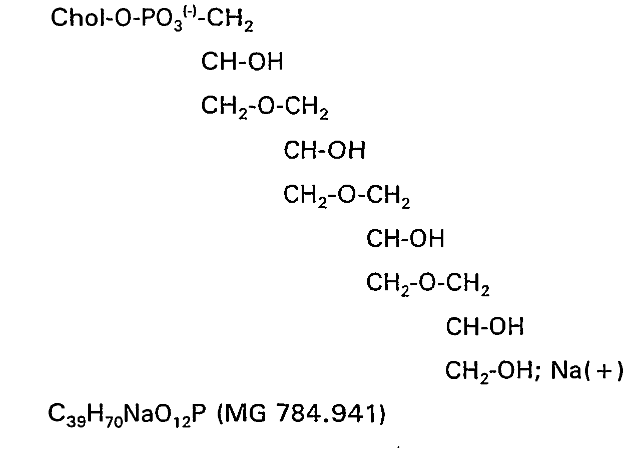

Weitere als geeignete Bestandteile d) verwendbare Verbindungen sind Alkylphosphoglycerine, Alkylphosphooligoglycerine, Alkylphosphoglycole, Alkylphosphopropandiole-(1.3) oder/und Alkylphosphopropandiole-(1.2). Die Alkylgruppe davon weist bevorzugt 13 bis 23 C-Atome auf, wobei vorzugsweise die Alkylgruppe der Verbindung der Komponente d) identisch mit der Gruppe R¹ der jeweils eingesetzten Komponente a) ist. Der Bestandteil d) liegt bevorzugt in einer Menge von 3 bis 50 Mol-%, insbesondere 5 bis 25 Mol-%, und mehr bevorzugt von 10 bis 20 Mol-% in den erfindungsgemäßen Liposomen vor.

Der Bestandteil d) liegt bevorzugt in einer Menge von 10 bis 20 Mol-% in den erfindungsgemäßen Liposomen vor.

Die Bestandteile a), b), c) und d), wie oben beschrieben, bilden zusammen bevorzugt 100 Mol-% der im Liposom enthaltenen Bestandteile.

Die wie vorstehend beschrieben zusammengesetzten Liposome weisen keinen eigenen Wirkstoffcharakter auf. Es handelt sich also um neutrale (im Sinne einer pharmazeutischen Wirksamkeit) Liposome, welche als Trägersysteme eingesetzt werden können. In einer weiteren bevorzugten Ausführungsform umfasst die Erfindung deshalb auch Liposome, wie oben beschrieben, welche weiterhin einen pharmazeutischen Wirkstoff in eingekapselter Form oder/und als weiteren Bestandteil der Liposomhülle enthalten. Für die Einkapselung kommt eine sehr breite Wirkstoffpalette in Betracht, beispielsweise Amphotericin B, Cyclosperin, pflanzliche Ceramide, sowie andere wirksame Verbindungen, wie beispielsweise Ether-Lysolecithine wie ET180CH3 (1-0-Octadecyl-2-0-methyl-glycero-3-phosphocholin). Bevorzugt werden solche Wirkstoffe eingeschlossen, welche eine polare Gruppe oder polare Bestandteile, beispielsweise OH-Gruppen oder Aminogruppen enthalten. Eine weitere Wirkstoffgruppe, welche vorteilhaft in die erfindungsgemäßen Liposome eingeschlossen werden kann, sind Alkylphosphocholine, insbesondere Phosphocholine mit einem Kohlenwasserstoffrest mit 12 bis 30, insbesondere 14 bis 24 C-Atomen, welcher gesättigt, einfach oder mehrfach ungesättigt sein kann. So wurde beispielsweise festgestellt, dass bei Verpackung von Hexadecylphosphocholin in die erfindungsgemäßen Liposomen bei intravenöser Gabe eine hervorragende Wirkung bei deutlich verminderter Toxizität auftritt. Auch bei Einschluss von Erucyl-Phosphocholin und Oleyl-Phosphocholin wurden Wirksamkeiten beobachtet, die jeweils deutlich oberhalb von oral verabreichten, nicht in Liposomen verkapselten freien Verbindungen liegt.

Durch Einschluss entsprechender Wirkstoffe können erfindungsgemäß insbesondere Arzneimittel zur Behandlung von Protozoenerkrankungen und zur Therapie von bakteriellen oder durch Pilzen hervorgerufenen Erkrankungen in Form von sterilfiltrierbaren oder/und hitzesterilisierbaren Liposomen bereitgestellt werden. Zusätzliche Wirkstoffe, welche in die Liposomen eingeschlossen werden können, umfassen bakterizide Wirkstoffe, wie etwa Oxytetracyclin, Doxycyclin oder Minocyclin, fungizide Wirkstoffe, wie etwa Amphotericin B oder Griseofulvin sowie immunsuprimierende Wirkstoffe, wie etwa Cyclosporin.

Beispielhafte Therapiemöglichkeiten sind wie folgt: Leishmaniasis unter Verwendung von Amphotericin B, Ehrlichniose unter Verwendung von Tetracyclinen, Pilzerkrankungen unter Verwendung von Amphotericin B und Immunsuppression unter Verwendung von Cyclosporin A als zusätzlichen Wirkstoff.

Ein wesentlicher Vorteil der erfindungsgemäßen Liposome besteht somit darin, dass bereits bei oraler Gabe eine deutlich überlegene Wirksamkeit gegenüber der oralen Gabe ohne Verpackung in die erfindungsgemäßen Liposome erzielt werden kann. Darüber hinaus ermöglicht die Verpackung in die erfindungsgemäßen Liposomen auch die Verabreichung in anderer Form, beispielsweise intravenös oder subkutan, wodurch oftmals die Wirksamkeit nochmals verbessert werden kann.

Die Erfindung umfasst weiterhin eine Zusammensetzung, welche die erfindungsgemäßen Liposome enthält. Diese Zusammensetzung besteht bevorzugt aus einer wässrigen Lösung, in der die erfindungsgemäßen Liposome dispergiert sind. Darüber hinaus kann die Zusammensetzung auch noch andere Lösungsmittel, insbesondere einen physiologisch verträglichen Alkohol enthalten. Bevorzugt sind wassermischbare Alkohole mit 2 bis 4 Kohlenstoffatomen, wie etwa Ethanol, 2-Propanol, 1,2-Propandiol und 2-Butanol oder Kombinationen davon.

Wie oben beschrieben, eignen sich die erfindungsgemäßen Liposome insbesondere zur Verwendung als Arzneimittelgrundlage zum Einbau von Wirkstoffen. Darüber hinaus betrifft die Erfindung auch Arzneimittel, welche die erfindungsgemäßen Liposome enthalten. Bevorzugt umfassen in einem solchen Arzneimittel die Liposome einen Wirkstoff in eingekapselter Form oder/und als weiteren Bestandteil der Liposomhülle.

Die erfindungsgemäßen Liposomen können auf einfache Weise durch Zusammenmischen der Bestandteile a), b), c) und d) hergestellt werden. Bevorzugt erfolgt das Vermischen in wässriger Lösung, wobei dem resultierenden Gemisch oder der wässrigen Lösung ein wassermischbarer, physiologisch annehmbarer Alkohol mit 2 bis 4 Kohlenstoffatomen hinzugefügt werden kann, sodass die Komponenten einen in Wasser dispergierten oder dispergierbaren Komplex bilden. Das Molverhältnis der (Ester)-Lysolecithinverbindung zu Alkohol beträgt bevorzugt 1:0,1 bis 1:500.

In der fertigen Liposomformulierung beträgt die Menge an (Ester)-Lysolecithin bevorzugt 0,1 bis 200 µMol/g.

Durch die einfache Löslichkeit der erfindungsgemäß zur Liposombildung eingesetzten Bestandteile ist kein Einsatz von Überdruck für die Herstellung der erfindungsgemäßen liposomalen Formlierungen notwendig. In der Regel genügt eine einfache Beschallung, unter Umständen reicht sogar Rühren aus. Damit vereinfacht und verbilligt sich das Herstellungsverfahren sehr. Außerdem können sterile Bedingungen durch das Lagern in entsprechend konzentrierten alkoholischen Lösungen problemlos eingehalten werden. Diese Vorteile gelten auch, wenn zusätzlich ein Wirkstoff der Formulierung einverleibt wird.

Unter Einsatz der erfindungsgemäßen Liposomen ist es weiterhin gelungen, ein Mittel gegen Lorenzos-Erkrankung (Lorenzos-Disease) herzustellen.

Dabei wurde Erucasäure in Lysolecithin oder Lecithin eingebaut und dann als Wirkstoff in die erfindungsgemäßen Liposomen eingekapselt. Die Erfindung betrifft somit auch die Verwendung der oben genannten Liposomen zur Herstellung eines Arzneimittels gegen Lorenzos-Erkrankung, wobei die Liposomen ein eingekapseltes Erucasäurederivat, insbesondere ein Erucasäure-Lysolecithin oder Erucasäure-Lecithin umfassen.

Die Erfindung wird durch die folgenden Beispiele weiter erläutert.

### Beispiel 1 - Herstellung der Ausgangsstoffe

Kommerziell erhältliches Cholesterin wurde durch Umkristallisation auf eine Reinheit > 98 % gereinigt.

1.2-Dioleoyl-sn-glycero-3-phosphocholin und andere Lecithine (Bestandteil c)) wurden nach im Stand der Technik beschriebenen Methoden hergestellt, ebenso wie die verwendeten Phosphatidylglycerine und Phosphatidyl-oligoglycerine.

Die Ester-Lysolecithine (Monoacylglycerophosphocholine) können auf einfache Weise aus Glycerophosphocholin hergestellt werden. Geeignete Lysolecithine umfassen z.B.:
1-Oleoyl-sn-glycero-3-phosphocholin
   (C₂₆H₅₂NO₇; 521.676)
1-Erucoyl-sn-glycero-3-phosphocholin
   (C₃₀H₆₀NO₇P; 577.784)
1-Linoleoyl-sn-glycero-3-phosphocholin
   (C₂₆H₅₀NO₇P; 519.660)
1 -Palmitoyl-sn-glycero-3-phosphocholin
   (C₂₄H₅₀NO₇P; 495.638)
1-Stearoyl-sn-glycero-3-phosphocholin
   (C₂₆H₅₄NO₇P; (523.692)
1-Oleoyl-sn-glycero-3-phospho-N.N.N.-trimethylpropylammonium
   (C₂₇H₅₄NO₇P; 535.703)
1-Erucoyl-sn-glycero-3-phospho-N.N.N.-trimethylpropylammonium
   (C₃₁H₆₂NO₇P; 591.81)
1-Linoleoyl-sn-glycero-3-phospho-N.N.N.-trimethyl-propylammonium
   (C₂₇H₅₂NO₇P; 533.687)
1-Palmitoyl-sn-glycero-3-phospho-N.N.N.-trimethyl-propylammonium
   (C₂₅H₅₂NO₇P; 509.687)
1-Stearoyl-sn-glycero-3-phospho-N.N.N.-trimethyl-propylammonium
   (C₂₇H₅₆NO₇P; 537.719)
1 -Oleoyl-sn-glycero-3-phospho-N.N.N.-trimethyl-butylammonium
   (C₂₈H₅₆NO₇P; 549.730)
1-Erucoyl-sn-glycero-3-phospho-N.N.N.-trimethyl-butylammonium
   (C₃₂H₆₄NO₇P; 605.838)

### Beispiel 2 - Herstellung von Liposomen

Es wurde festgestellt, dass Ester-Lysolecithine aufgrund ihrer ausgezeichneten dispergierenden Eigenschaften hervorragend geeignet sind, um Lipidgemische vollständig in liposomale Dispersionen umzuwandeln. Dabei erfolgt die Bildung der Liposomen unter milden Bedingungen, beispielsweise durch einfaches Beschallen in einem Ultraschallbad.

Die Lipidgemische können pharmazeutische Wirkung besitzen, z.B. im Falle von Erucasäurederivaten zur Behandlung der X-Adrenoleukodystrophie oder durch Einbau von Wirkstoffen, wie beispielsweise Amphotericin C, Cyclosporin usw., als Arzneimittel Verwendung finden.

Zur Herstellung von 1000 ml einer liposomalen Dispersion aus den Lipidgemischen in einer Endkonzentration von 60 bis 100 mM an Gesamtlipid wird wie folgt vorgegangen:

| | Oleoyl-GPC | 0,0 - GPC | Cholesterin | 0,0-GPG |
|---|---|---|---|---|
| | (521,68) | (786,13) | (386,66) | (797,03) |
| | (Oleoyl-Glycero-phosphocholin) | 1,2-Dioleoyl-glycerophosphocholin | | 1,2-Dioleoyl-sn glycero-3 phosphoglycerin-mononatriumsalz |
| A - | a) 45,0 | - | 47,5 | 7,5→100% |
| | b) 45,0 | - | 47,5 | 7,5 → 100 mM |
| | c) 23,48 | - | 18,37 | 5,98 → 43,83 g |
| Die Dispersion ist 100 mM an Lipid und enthält ~ 4,8 % Lipide. | | | | |
| B- | a) 40,0 | 10,0 | 45,0 | 5,0 → 100 % |
| | b) 40,0 | 10,0 | 45,0 | 5,0 → 100 mM |
| | c) 20,87 | 7,86 | 14,40 | 3,99→47,12 g |
| Die Dispersion ist 100 mM an Lipid und enthält ~ 4,7 % Lipide. | | | | |
| C - | a) 35,0 | 15,0 | 40,0 | 10,0→100 % |
| | b) 35,0 | 15,0 | 40,0 | 10,0→100 mM |
| | c) 18,26 | 11,79 | 15,47 | 7,97→53,49 g |
| Die Dispersion ist 100 mM an Lipid und enthält ~ 5,4 % Lipide. | | | | |
| D - | a) 30,0 | 20,0 | 40,0 | 10,0 → 100 % |
| | b) 30,0 | 20,0 | 40,0 | 10,0→100 mM |
| | c) 15,65 | 15,72 | 15,47 | 7,97→54,81 g |
| Die Dispersion ist 100 mM an Lipid und enthält ~ 5,5 % Lipide. | | | | |
| E - | a) 25,0 | 25,0 | 40,0 | 10,0 → 100 % |
| | b) 22,5 | 22,5 | 36,0 | 9,0 → 90 mM |
| | c) 17,74 | 17,69 | 13,92 | 7,17→56,52 g |
| Die Dispersion ist 90 mM an Lipid und enthält ~ 5,7 % Lipide. | | | | |
| F - | a) 20,0 | 35,0 | 30,0 | 15,0→100 % |
| | b) 16,0 | 28,0 | 24,0 | 12,0 → 80 mM |
| | c) 8,35 | 22,01 | 9,28 | 9,56 → 49,2 g |
| Die Dispersion ist 80 mM an Lipid und enthält ~ 4,9 % Lipide. | | | | |
| G - | a) 15,0 | 35,0 | 35,0 | 15,0 → 100 % |
| | b) 12,0 | 28,0 | 28,0 | 12,0 → 80 mM |
| | c) 6,26 | 22,01 | 10,83 | 9,56→48,66 g |
| Die Dispersion ist 80 mM an Lipid und enthält ~ 4,9 % Lipide. | | | | |

### Beispiel 3 - Zusammensetzung

| | | molare Anteile |
|---|---|---|
| (Ester)-Lysolecithine | | 15-90 % |
| Lecithine | | 0-40 % |
| Cholesterin | | 0-60 % |
| neg. | Ladungsträger | 3-50 % |
| | | |
| z.B. | Dipalmitoyl- | |
| | Distearoyl- | sn-G-3-phosphoglycerine oder -oligo- |
| | | glycerine |
| | Dioleoyl- | |
| | | |
| z.B. | 1-Palmitoyl- | |
| | 1-Stearoyl- | sn-G-3-phosphoglycerin |
| | 1-Oleoyl- | |
| | | |
| z.B. | Cholesterin-phospho-glycerine oder -oligoglycerine. | |

Dabei wird bevorzugt beachtet, dass die Systeme zusammen passen, also in einer Formulierung ganz besonders bevorzugt nur ein einziger Fettsäurebestandteil verwendet wird.

| | Einwaage |
|---|---|
| | (mMol/l = mM) |
| 1-Stearoyl-sn-glycero-3-phosphocholin | |
| 1-S-G-3-PC | 35 |
| Cholesterin | 40 |
| 1-S-G-3-P-diG | 6 |
| | 79 |
| | |
| 1-S-G-3-PC | 35 |
| Cholesterin | 40 |
| 1-S-G-3-P-diG | 15 |
| | 90 |
| | |
| 1-S-G-3-PC | 40 |
| Cholesterin | 40 |
| Chol-P-diG | 10 |
| | 90 |

Wichtig sind aber auch Formulierungen, die (Ether)-Lysolecithine enthalten, die keine Wirkstoffqualität im Sinne einer Antitumorwirkung oder Antiparasitenwirkung besitzen. Diese Formulierungen sind deshalb besonders wichtig, da sie hitzesterilisierbar sind und deshalb besonders einfach gehandhabt werden können, zum Beispiel:

| 1-Octadecyl-sn-glycero-3-phosnhocholin | |
|---|---|
| 1-C_{18:0}-sn-G-3-PC | 35 |
| Cholesterin | 45 |
| 1-C_{18:0}-sn-G-3-P-diG | 5 |
| | 85 |
| | |
| 1-C_{18:0}-sn-G-3-PC | 35 |
| Cholesterin | 40 |
| Chol-PG | 10 |
| | 85 |

### Entsprechend:

| 1-Oleyl-sn-glycero-3-phosphocholin | |
|---|---|
| 1-C_{18:1}-sn-G-3-PC | 40 |
| Cholesterin | 45 |
| 1-C_{18:1}-sn-G-3-PG | 5 |
| | 90 |
| | |
| 1-C_{18:1}-sn-G-3-PC | 45 |
| Cholesterin | 45 |
| Cholesterin-PG | 10 |
| | 110 |

| 1-Erucyl-sn-glycero-3-phosphocholin | |
|---|---|
| 1-C_{22:1}-sn-G-3-PC | 70 |
| Cholesterin | 15 |
| 1-C_{22:1}-sn-G-3-diG | 15 |
| | 100 |
| | |
| 1-C_{22:1}-sn-G-3-PC | 80 |
| Chol-P-diG | 20 |
| | 100 |

## Patentansprüche

1. Liposom enthaltend
a) 10 bis 50 Mol-% (Ester)-Lysolecithin der Formel I 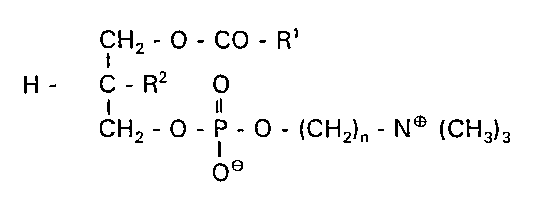 in der
R¹ ein Kohlenwasserstoffrest mit 13 bis 23 C-Atomen ist,
R² H, OH oder OR³ darstellt, worin R³ ein C₁-C₃-Alkyl- oder ein Allylrest ist, und
n 2, 3 oder 4 bedeutet
b) 10 bis 50 Mol-% Cholesterin,
c) 10 bis 50 Mol-% Lecithin und
d) 5 bis 25 Mol-% eines negativen Ladungsträgers, ausgewählt aus der Gruppe bestehend aus Phosphatidylmono-, -di-, -tri- und -tetraglycerinen, Cholesterinphosphomono- und -oligoglycerinen.

2. Liposom nach Anspruch 1,
**dadurch gekennzeichnet,**
**dass** R¹ in Formel I einen C₁₃-C₁₉-Alkylrest, einen C₁₅-C₂₃-Alkenylrest, einen C₁₅-C₂₃-Alkadienylrest oder einen C₁₅-C₂₃-Alkatrienylrest darstellt.

3. Liposom nach Anspruch 1 oder 2,
**dadurch gekennzeichnet,**
**dass** n in Formel I die Zahl 2 bedeutet.

4. Liposom nach einem der vorhergehenden Ansprüche,
**dadurch gekennzeichnet,**
**dass** es weiterhin einen Wirkstoff in eingekapselter Form oder/und als weiteren Bestandteil der Liposomenhülle enthält.

5. Zusammensetzung enthaltend Liposome nach einem der Ansprüche 1 bis 4.

6. Arzneimittel enthaltend Liposome nach einem der Ansprüche 1 bis 4, gegebenenfalls mit einem pharmakologisch geeigneten Träger- oder Verdünnungsmittel.

7. Arzneimittel nach Anspruch 6,
**dadurch gekennzeichnet,**
**dass** es ein Liposom nach einem der Ansprüche 1 bis 4 und darin eingekapselt einen Wirkstoff umfasst.

8. Arzneimittelgrundlage zum Einbau von Wirkstoffen, umfassend Liposomen nach einem der Ansprüche 1 bis 3.

9. Arzneimittel nach einem der Ansprüche 6 oder 7 oder Arzneimittelgrundlage nach Anspruch 8,
**dadurch gekennzeichnet,**
**dass** sie in einer zur oralen, intravenösen oder subkutanen Verabreichung geeigneten Form vorliegt.

10. Verfahren zur Herstellung von Liposomen nach einem der Ansprüche 1 bis 4,
**dadurch gekennzeichnet,**
**dass** man
a) 10 bis 50 Mol-% (Ester)-Lysolecithin der Formel 1 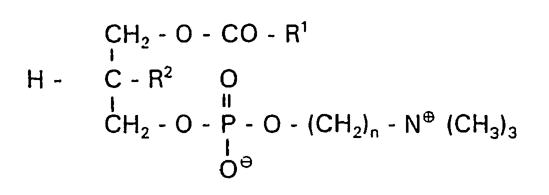 in der
R¹ ein Kohlenwasserstoffrest mit 13 bis 23 C-Atomen ist,
R² H, OH oder OR³ darstellt, worin R³ ein C₁-C₃-Alkyl- oder Allylrest ist, und
n 2, 3 oder 4 bedeutet
b) 10 bis 50 Mol-% Cholesterin,
c) 10 bis 50 Mol-% Lecithin und
d) 5 bis 25 Mol-% eines negativen Ladungsträgers, ausgewählt aus der Gruppe bestehend aus Phosphatidylmono-, -di-, -tri- und -tetraglycerinen und Cholesterinphosphomono- und - oligoglycerinen vermischt.

11. Verwendung von Liposomen nach einem der Ansprüche 1 bis 4 zur Herstellung eines Arzneimittels gegen Lorenzos-Erkrankungen, wobei die Liposomen ein eingekapseltes Erucasäurederivat, insbesondere ein Erucasäure-Lysolecithin oder Erucasäurelecithin umfassen.

12. Liposom enthaltend
a) 10 bis 90 Mol-% (Ester)-Lysolecithin der Formel I 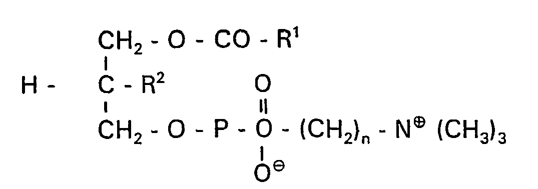 in der
R¹ ein Kohlenwasserstoffrest mit 13 bis 23 C-Atomen ist,
R² H, OH oder OR³ darstellt, worin R³ ein C₁-C₃-Alkyl- oder ein Allylrest ist, und
n 2, 3 oder 4 bedeutet
b) 0 bis 60 Mol-% Cholesterin,
c) 0 bis 50 Mol-% Lecithin und
d) 3 bis 50 Mol-% eines negativen Ladungsträgers, ausgewählt aus der Gruppe bestehend aus Phosphatidylmono-, -di-, -tri- und -tetraglycerinen, Cholesterinphosphomono- und -oligoglycerinen und/oder Alkylphosphoglycerinen, Alkylphosphooligoglycerinen, Alkylphosphoglycolen, Alkylphosphopropandiolen-(1.3) oder/und Alkylphosphopropandiolen-(1.2).

13. Liposom nach Anspruch 12,
**dadurch gekennzeichnet,**
**dass** es ein Merkmal nach einem der Ansprüche 1 bis 4 aufweist.

14. Zusammensetzung enthaltend Liposome nach einem der Ansprüche 12 oder 13.

15. Arzneimittel enthaltend Liposome nach einem der Ansprüche 12 oder 13, gegebenenfalls mit einem pharmakologisch geeigneten Träger- oder Verdünnungsmittel.

16. Arzneimittel nach Anspruch 15,
**dadurch gekennzeichnet,**
**dass** es ein Liposom nach einem der Ansprüche 12 oder 13 und darin eingekapselt einen Wirkstoff umfasst.

17. Arzneimittelgrundlage zum Einbau von Wirkstoffen, umfassend Liposomen nach einem der Ansprüche 12 oder 13.

18. Verfahren zur Herstellung von Liposomen nach einem der Ansprüche 12 oder 13,
**dadurch gekennzeichnet,**
**dass** man
a) 10 bis 90 Mol-% (Ester)-Lysolecithin der Formel I 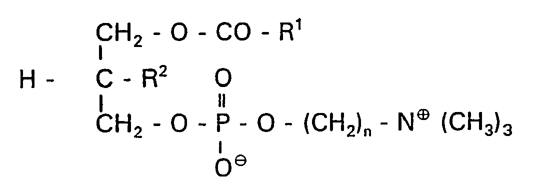 in der
R¹ ein Kohlenwasserstoffrest mit 13 bis 23 C-Atomen ist,
R² H, OH oder OR³ darstellt, worin R³ ein C₁-C₃-Alkyl- oder ein Allylrest ist, und
n 2, 3 oder 4 bedeutet
b) 0 bis 60 Mol-% Cholesterin,
c) 0 bis 50 Mol-% Lecithin und
d) 3 bis 50 Mol-% eines negativen Ladungsträgers, ausgewählt aus der Gruppe bestehend aus Phosphatidylmono-, -di-, -tri- und -tetraglycerinen, Cholesterinphosphomono- und -oligoglycerinen und/oder Alkylphosphoglycerinen, Alkylphosphooligoglycerinen, Alkylphosphoglycolen, Alkylphosphopropandiolen-(1.3) oder/und Alkylphosphopropandiolen-(1.2) vermischt.

19. Verwendung von Liposomen nach einer der Ansprüche 12 oder 13 zur Herstellung eines Arzneimittels gegen Lorenzos-Erkrankungen, wobei die Liposomen ein eingekapseltes Erucasäurederivat, insbesondere ein Erucasäure-Lysolecithin oder Erucasäurelecithin, umfassen.

## Claims

1. Liposome containing
a) 10 to 50 mole % (ester)-lysolecithin of formula I 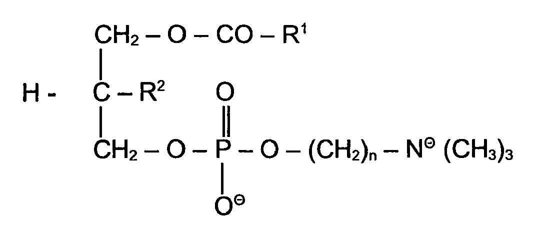 in which
R¹ is a hydrocarbon residue with 13 to 23 C atoms,
R² represents H, OH or OR³ in which R³ represents a C₁-C₃ alkyl or an allyl residue and
n denotes 2, 3 or 4
b) 10 to 50 mole % cholesterol,
c) 10 to 50 mole % lecithin and
d) 5 to 25 mole % of a negative charge carrier selected from the group comprising phosphatidylmono, -di-, -tri-, and -tetraglycerols and cholesterol phosphomonoglycerols and cholesterol phosphooligoglycerols.

2. Liposome as claimed in claim 1,
**characterized in that**
R¹ in formula I represents a C₁₃-C₁₉ alkyl residue, a C₁₅-C₂₃ alkenyl residue, a C₁₅-C₂₃ alkadienyl residue or a C₁₅-C₂₃ alkatrienyl residue.

3. Liposome as claimed in claim 1 or 2,
**characterized in that**
n in formula I denotes the number 2.

4. Liposome as claimed in one of the previous claims,
**characterized in that**
it additionally contains an active substance in an encapsulated form or/and as a further component of the liposomal coat.

5. Composition containing liposomes as claimed in one of the claims 1 to 4.

6. Pharmaceutical preparation containing liposomes as claimed in one of the claims 1 to 4, optionally together with a pharmacologically suitable carrier medium or diluent.

7. Pharmaceutical preparation as claimed in claim 6,
**characterized in that**
it comprises a liposome as claimed in one of the claims 1 to 4 in which an active substance is encapsulated.

8. Pharmaceutical base for incorporating active substances comprising liposomes as claimed in one of the claims 1 to 3.

9. Pharmaceutical preparation as claimed in one of the claims 6 or 7 or pharmaceutical base as claimed in claim 8,
**characterized in that**
it is present in a suitable form for oral, intravenous or subcutaneous administration.

10. Process for producing liposomes as claimed in one of the claims 1 to 4,
**characterized in that**
a) 10 to 50 mole % (ester)-lysolecithin of formula I 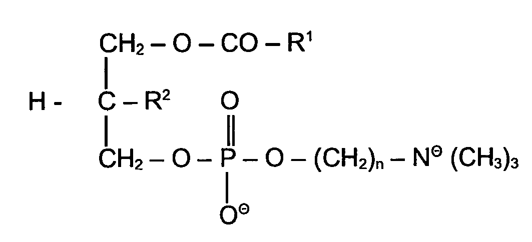 in which
R¹ is a hydrocarbon residue with 13 to 23 C atoms,
R² represents H, OH or OR³ in which R³ represents a C₁-C₃ alkyl or allyl residue and
n denotes 2, 3 or 4
b) 10 to 50 mole % cholesterol,
c) 10 to 50 mole % lecithin and
d) 5 to 25 mole % of a negative charge carrier selected from the group comprising phosphatidylmono, -di-, -tri-, and -tetraglycerols and cholesterol phosphomonoglycerols and cholesterol phosphooligoglycerols are mixed.

11. Use of liposomes as claimed in one of the claims 1 to 4 for producing a pharmaceutical preparation against Lorenzo's diseases in which the liposomes comprise an encapsulated erucic acid derivative and in particular an erucic acid-lysolecithin or erucic acid-lecithin.

12. Liposome containing
a) 10 to 90 mole % (ester)-lysolecithin of formula I 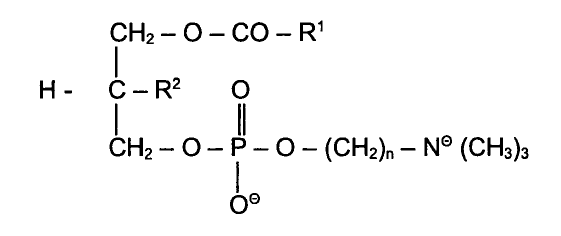 in which
R¹ is a hydrocarbon residue with 13 to 23 C atoms,
R² represents H, OH or OR³ in which R³ represents a C₁-C₃ alkyl or an allyl residue and
n denotes 2, 3 or 4
b) 0 to 60 mole % cholesterol,
c) 0 to 50 mole % lecithin and
d) 3 to 50 mole % of a negative charge carrier selected from the group comprising phosphatidylmono, -di-, -tri-, and -tetraglycerols and cholesterol phosphomonoglycerols and cholesterol phosphooligoglycerols and/or alkylphosphoglycerols, alkylphosphooligoglycerols, alkylphosphoglycols, alkylphosphopropanediols-(1,3) or/and alkylphosphopropanediols-(1,2).

13. Liposome as claimed in claim 12,
**characterized in that**
it has a feature as claimed in one of the claims 1 to 4.

14. Composition containing liposomes as claimed in one of the claims 12 or 13.

15. Pharmaceutical preparation containing liposomes as claimed in one of the claims 12 or 13, optionally together with a pharmacologically suitable carrier medium or diluent.

16. Pharmaceutical preparation as claimed in claim 15,
**characterized in that**
it comprises a liposome as claimed in one of the claims 12 or 13 in which an active substance is encapsulated.

17. Pharmaceutical base for incorporating active substances comprising liposomes as claimed in one of the claims 12 or 13.

18. Process for producing liposomes as claimed in one of the claims 12 or 13,
**characterized in that**
a) 10 to 90 mole % (ester)-lysolecithin of formula I 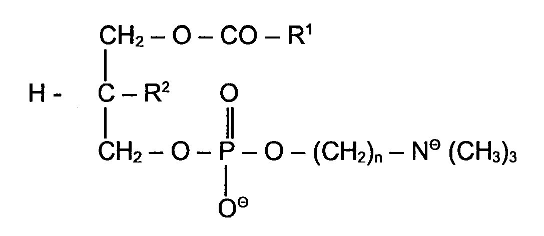 in which
R¹ is a hydrocarbon residue with 13 to 23 C atoms,
R² represents H, OH or OR³ in which R³ represents a C₁-C₃ alkyl or an allyl residue and
n denotes 2, 3 or 4
b) 0 to 60 mole % cholesterol,
c) 0 to 50 mole % lecithin and
d) 3 to 50 mole % of a negative charge carrier selected from the group comprising phosphatidylmono, -di-, -tri-, and -tetraglycerols and cholesterol phosphomonoglycerols and cholesterol phosphooligoglycerols and/or alkylphosphoglycerols, alkylphosphooligoglycerols, alkylphosphoglycols, alkylphosphopropanediols-(1,3) or/and alkylphosphopropanediols-(1,2) are mixed.

19. Use of liposomes as claimed in one of the claims 12 or 13 for the production of a pharmaceutical preparation against Lorenzo's diseases in which the liposomes comprise an encapsulated erucic acid derivative and in particular a erucic acid-lysolecithin or erucic acid-lecithin.

## Revendications

1. Liposome contenant
a) 10 à 50 mol% de (ester)-lysolécithine de formule I 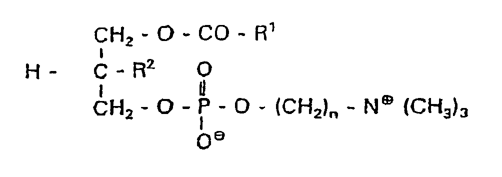 où
R¹ est un groupement hydrocarboné avec 13 à 23 atomes C,
R² représente H, OH ou OR³, où R³ est un groupement C₁-C₃-alkyle ou un groupement allyle, et
n signifie 2, 3 ou 4
b) 10 à 50 mol% de cholestérol,
c) 10 à 50 mol% de lécithine et
d) 5 à 25 mol% d'un porteur de charge négative, choisi dans le groupe consistant en les phosphatidylmono-, di-, tri- et tétraglycérines, les cholestérolphosphomono- et oligoglycérines.

2. Liposome selon la revendication 1 **caractérisé en ce que** R¹ dans la formule I représente un groupement C₁₃-C₁₉-alkyle, un groupement C₁₅-C₂₃₋alcényle, un groupement C₁₅-C₂₃-alcadiényle ou un groupement C₁₅-C₂₃₋alcatriényle.

3. Liposome selon la revendication 1 ou 2 **caractérisé en ce que** n dans la formule I signifie le chiffre 2.

4. Liposome selon l'une des revendications précédentes **caractérisé en ce qu'**il contient en outre un principe actif sous forme encapsulée et/ou comme constituant supplémentaire de l'enveloppe du liposome.

5. Composition contenant des liposomes selon l'une des revendications 1 à 4.

6. Médicament contenant des liposomes selon l'une des revendications 1 à 4, éventuellement avec un support ou diluant pharmacologiquement approprié.

7. Médicament selon la revendication 6 **caractérisé en ce qu'**il comprend un liposome selon l'une des revendications 1 à 4 et un principe actif encapsulé dans celui-ci.

8. Base de médicament destinée à l'incorporation de principes actifs, comprenant des liposomes selon l'une des revendications 1 à 3.

9. Médicament selon l'une des revendications 6 ou 7 ou base de médicament selon la revendication 8 **caractérisé en ce qu'**il est présent dans une forme appropriée à l'administration orale, intraveineuse ou sous-cutanée.

10. Procédé de production de liposomes selon l'une des revendications 1 à 4 **caractérisé en ce que** l'on mélange
a) 10 à 50 mol% de (ester)-lysolécithine de formule I 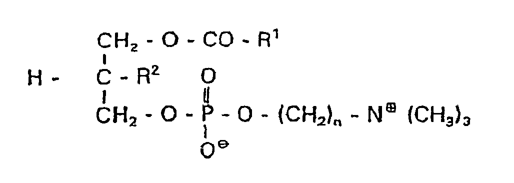 où
R¹ est un groupement hydrocarboné avec 13 à 23 atomes C,
R² représente H, OH ou OR³, où R³ est un groupement C₁-C₃-alkyle ou un groupement allyle, et
n signifie 2, 3 ou 4
b) 10 à 50 mol% de cholestérol,
c) 10 à 50 mol% de lécithine et
d) 5 à 25 mol% d'un porteur de charge négative, choisi dans le groupe consistant en les phosphatidylmono-, di-, tri- et tétraglycérines, les cholestérolphosphomono- et oligoglycérines.

11. Utilisation de liposomes selon l'une des revendications 1 à 4 pour la production d'un médicament contre les maladies de Lorenzos où les liposomes comprennent un dérivé de l'acide érucique encapsulé, en particulier une lysolécithine d'acide érucique ou une lécithine d'acide érucique.

12. liposome contenant
a) 10 à 90 mol% de (ester)-lysolécithine de formule I 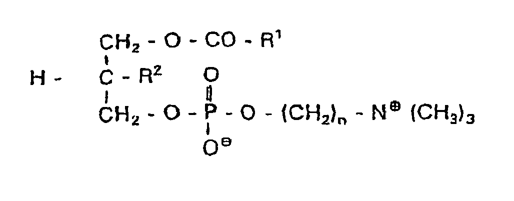 où
R¹ est un groupement hydrocarboné avec 13 à 23 atomes C,
R² représente H, OH ou OR³, où R³ est un groupement C₁-C₃-alkyle ou un groupement allyle, et
n signifie 2, 3 ou 4
b) 0 à 60 mol% de cholestérol,
c) 0 à 50 mol% de lécithine et
d) 3 à 50 mol% d'un porteur de charge négative, choisi dans le groupe consistant en les phosphatidylmono-, di-, tri- et tétraglycérines, les cholestérolphosphomono- et oligoglycérines et/ou les alkylphosphoglycérines, les alkylphosphooligoglycérines, les alkylphosphoglycols, les alkylphosphopropanediols-(1.3) et/ou les alkylphosphopropanediols-(1.2).

13. Liposome selon la revendication 12 **caractérisé en ce qu'**il présente une caractéristique selon l'une des revendications 1 à 4.

14. Composition contenant des liposomes selon l'une des revendications 12 ou 13.

15. Médicament contenant des liposomes selon l'une des revendications 12 ou 13, éventuellement avec un support ou diluant pharmacologiquement approprié.

16. Médicament selon la revendication 15 **caractérisé en ce qu'**il comprend un liposome selon l'une des revendications 12 ou 13 et un principe actif encapsulé dans celui-ci.

17. Base de médicament destinée à l'incorporation de principes actifs, comprenant des liposomes selon l'une des revendications 12 ou 13.

18. Procédé de production de liposomes selon l'une des revendications 12 ou 13 **caractérisé en ce que** l'on mélange
a) 10 à 90 mol% de (ester)-lysolécithine de formule 1 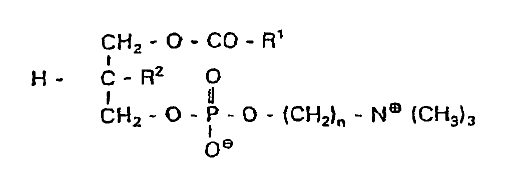 où
R¹ est un groupement hydrocarboné avec 13 à 23 atomes C,
R² représente H, OH ou OR³, où R³ est un groupement C₁-C₃-alkyle ou un groupement allyle, et
n signifie 2, 3 ou 4
b) 0 à 60 mol% de cholestérol,
c) 0 à 50 mol% de lécithine et
d) 3 à 50 mol% d'un porteur de charge négative, choisi dans le groupe consistant en les phosphatidylmono-, di-, tri- et tétraglycérines, les cholestérolphosphomono- et oligoglycérines et/ou les alkylphosphoglycérines, les alkylphosphooligoglycérines, les alkylphosphoglycols, les alkylphosphopropanediols-(1.3) et/ou les alkylphosphopropanediols-(1.2).

19. Utilisation de liposomes selon l'une des revendications 12 ou 13 pour la production d'un médicament contre les maladies de Lorenzos où les liposomes comprennent un dérivé de l'acide érucique encapsulé, en particulier une lysolécithine d'acide érucique ou une lécithine d'acide érucique.
